# EUROPEAN PATENT SPECIFICATION

(11) **EP 2 894 146 B1**
(45) Date of publication and mention of the grant of the patent: **22.08.2018**
(21) Application number: 13835371.9
(22) Date of filing: 26.02.2013
(51) Int. Cl.: C07C 311/48, H01G 9/022, C07C 303/40, H01G 11/54, H01G 11/64

(54) **METHOD FOR PREPARING BIS(FLUOROSULFONYL)IMIDE**
VERFAHREN ZUR HERSTELLUNG VON BIS(FLUORSULFONYL)IMID
PROCÉDÉ DE PRÉPARATION DE BIS(FLUOROSULFONYL)IMIDE

(30) Priority: 10.09.2012 CN 201210331995
(43) Date of publication of application: 15.07.2015
(73) Proprietor: HSC Corporation, Zhangjiagang, Jiangsu 215635 (CN)
(72) Inventor: SHEN, Ming, Zhangjiagang Jiangsu 215635 (CN); SHEN, Jinliang, Zhangjiagang Jiangsu 215635 (CN); ZHANG, Xianlin, Zhangjiagang Jiangsu 215635 (CN); YANG, Zhiyong, Zhangjiagang Jiangsu 215635 (CN); ZHANG, Liya, Zhangjiagang Jiangsu 215635 (CN); WU, Guodong, Zhangjiagang Jiangsu 215635 (CN)
(74) Representative: Capasso, Olga
(86) International application number: PCT/CN2013/071868
(87) International publication number: WO 2014/036814

(56) References cited:
- WO-A1-2009/123328
- CA-A1- 2 527 802
- CN-A- 102 046 523
- CN-A- 102 405 189
- US-A1- 2004 097 757
- US-A1- 2012 041 233
- US-B2- 7 253 317

## Description

### Technical Field

The invention relates to a method of preparing lithium bis(fluorosulfonyl)imide.

### Background Art

The lithium bis(fluorosulfonyl)imide referred to herein is a compound having the following structure:

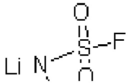

Lithium bis(fluorosulfonyl)imide may be used in a variety of fields. It may be used as an electrolyte additive of a lithium ion cell for improving the cyclic performance of the lithium ion cell; as an electrolyte of a primary cell; as a catalyst for a polymerization reaction; or as an antistatic agent in the industrial field.

The research shows nowadays that, when used in the electrolyte of a rechargeable lithium cell, lithium bis(fluorosulfonyl)imide can decrease effectively the low temperature resistance of the SEI layer formed on the surface of the electrode plate, and decrease the capacity loss of the lithium cell during storage, so as to provide a high capacity cell and increase the electrochemical properties of the cell.

The techniques for preparing lithium bis(fluorosulfonyl)imide that can be found in both domestic and foreign reports mainly include the reaction of bis(chlorosulfonyl)imide as a starting material with fluorosulfonic acid to obtain bis(fluorosulfonyl)imide which is then reacted with a lithium salt to obtain lithium bis(fluorosulfonyl)imide, and the replacement reaction of lithium perchlorate as the starting material with potassium bis(fluorosulfonyl)imide, as proposed by Beran, et al, to obtain the lithium bis(fluorosulfonyl)imide product. According to US7253317, an alkali metal fluoride and bis(chlorosulfonyl)imide are used to synthesize an alkali metal salt of bis(fluorosulfonyl)imide in nitromethane. However, the product comprises impurities such as chlorofluorosulfonyl imide, fluorosulfonyl imide, etc, which are difficult to be separated, leading to a low yield. Moreover, a solid salt is difficult to be obtained. The above process routes have such disadvantages as long length, complex product composition, difficult separation of acid esters or potassium ions and the like entrained in the product, which affect the industrial implementation of the product.

Against the above disadvantages, lithium bis(chlorosulfonyl)imide is used as a starting material directly in the present invention to carry out fluorine-chlorine exchange with an alkali metal fluoride directly in the presence of a catalyst and a suitable solvent to obtain solid lithium bis(fluorosulfonyl)imide. The ion contents of fluorine, chlorine, potassium, etc in the product are all below 10ppm and fully meet the requirement of its use in the electrolyte material of a lithium cell. Additionally, the process route is simple, and industrial manufacture can be realized easily.

### Summary

In order to overcome the problems existing in the prior art, the inventors of the invention have conducted intensive study and have found a new process suitable for preparing lithium bis(fluorosulfonyl)imide, comprising: direct fluorine-chlorine exchange of lithium bis(chlorosulfonyl)imide used as a starting material with an alkali metal fluoride in the presence of a suitable catalyst and a suitable solvent to obtain a lithium bis(fluorosulfonyl)imide product. Compared with the processes generally used across the world, the process route of the preparation method according to the invention is simple and can be controlled easily, with less than 100ppm or even less than 10ppm of fluorine, chlorine, alkali metal ions and other impurities entrained in the product, meeting the requirement of electronic application.

In one aspect, the invention provides a method of preparing lithium bis(fluorosulfonyl)imide, comprising: reacting lithium bis(chlorosulfonyl)imide with an alkali metal fluoride selected from lithium fluoride, potassium fluoride, sodium fluoride or mixtures thereof used as a fluorinating agent in a solvent selected from saturated alkyl carbonate and/or saturated fluoroalkyl carbonate in the presence of a crown ether phase transfer catalyst adaptive to the alkali metal fluoride to obtain lithium bis(fluorosulfonyl)imide,
wherein lithium bis(chlorosulfonyl)imide has the following structure: 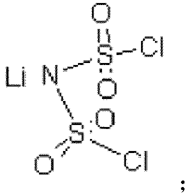 and
lithium bis(fluorosulfonyl)imide product has the following structure:

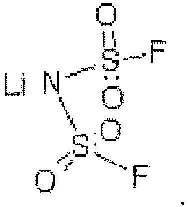

In an embodiment of the invention the amount of the alkali metal fluoride added is 2.01-10 times the moles of lithium bis(chlorosulfonyl)imide.

In a preferred embodiment of the invention, the saturated alkyl carbonate solvent is selected from dimethyl carbonate, diethyl carbonate, dipropyl carbonate, methyl ethyl carbonate, methyl propyl carbonate, ethyl propyl carbonate or mixtures thereof, and added in an amount 1-10 times the mass fraction of lithium bis(chlorosulfonyl)imide.

In a preferred embodiment of the invention, the saturated fluoroalkyl carbonate solvent is selected from trifluoroethyl ethyl carbonate, bis(trifluoroethyl) carbonate, trifluoroethyl methyl carbonate, or mixtures thereof, and added in an amount 1-10 times the mass fraction of lithium bis(chlorosulfonyl)imide.

In a preferred embodiment of the invention, the crown ether phase transfer catalyst adaptive to the alkali metal fluoride is 12-crown-4(lithium fluoride), 15-crown-5(sodium fluoride) or 18-crown-6(potassium fluoride).

In a preferred embodiment of the invention, the amount of the crown ether phase transfer catalyst added is 0.1%-10% of the mass fraction of lithium bis(chlorosulfonyl)imide.

In an embodiment, the reaction of the invention is carried out at a temperature of 40-130°C and the reaction time is 8-24 hours.

Particularly, the invention provides a method of preparing lithium bis(fluorosulfonyl)imide, comprising: reacting lithium bis(chlorosulfonyl)imide with an alkali metal fluoride used as a fluorinating agent in a solvent of saturated alkyl carbonate and/or saturated fluoroalkyl carbonate in the presence of a crown ether phase transfer catalyst adaptive to the alkali metal fluoride to obtain lithium bis(fluorosulfonyl)imide product.

In the invention, the reaction formula is as follows: 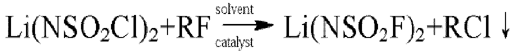 wherein R is selected from one or more of lithium, potassium, sodium.

According to the invention, the alkali metal fluoride RF is selected from lithium fluoride, potassium fluoride, sodium fluoride or mixtures thereof, wherein the amount of the alkali metal fluoride added is 2.01-10 times the moles of lithium bis(chlorosulfonyl)imide.

In an embodiment of the invention, the saturated alkyl carbonate solvent is selected from dimethyl carbonate, diethyl carbonate, dipropyl carbonate, methyl ethyl carbonate, methyl propyl carbonate, ethyl propyl carbonate or mixtures thereof, and added in an amount 1-10 times the mass fraction of lithium bis(chlorosulfonyl)imide.

In another embodiment of the invention, the saturated fluoroalkyl carbonate solvent is selected from trifluoroethyl ethyl carbonate, bis(trifluoroethyl) carbonate, trifluoroethyl methyl carbonate, or mixtures thereof, and added in an amount 1-10 times the mass fraction of lithium bis(chlorosulfonyl)imide.

According to the invention, the saturated alkyl carbonate solvent may be mixed with the saturated fluoroalkyl carbonate solvent and used as a solvent in the reaction of the invention.

According to the invention, the crown ether phase transfer catalyst adaptive to the alkali metal fluoride is preferably 12-crown-4(lithium fluoride), 15-crown-5(sodium fluoride) or 18-crown-6(potassium fluoride). That's to say, the crown ether phase transfer catalyst adaptive to the alkali metal fluoride is preferably 12-crown-4(lithium fluoride) adaptive to lithium fluoride, 15-crown-5(sodium fluoride) adaptive to sodium fluoride or 18-crown-6(potassium fluoride) adaptive to potassium fluoride.

The 12-crown-4(lithium fluoride) used herein means that the crown ether phase transfer catalyst adaptive to the alkali metal fluoride is 12-crown-4 when the alkali metal fluoride used is lithium fluoride. The 15-crown-5(sodium fluoride) used herein means that the crown ether phase transfer catalyst adaptive to the alkali metal fluoride is 15-crown-5 when the alkali metal fluoride used is sodium fluoride. The 18-crown-6(potassium fluoride) used herein means that the crown ether phase transfer catalyst adaptive to the alkali metal fluoride is 18-crown-6 when the alkali metal fluoride used is potassium fluoride.

According to the invention, the amount of the crown ether phase transfer catalyst added is 0.1%-10% of the mass fraction of lithium bis(chlorosulfonyl)imide.

According to the invention, the reaction is carried out at a temperature of 40-130°C and the reaction time is 8-24 hours.

According to the invention, the object of the invention is achieved specifically in the following way:
Lithium bis(chlorosulfonyl)imide used as a starting material and alkali metal fluoride used as a fluorinating agent react at a temperature in a solvent of saturated alkyl carbonate and/or saturated fluoroalkyl carbonate in the presence of a crown ether phase transfer catalyst adaptive to the alkali metal fluoride to obtain lithium bis(fluorosulfonyl)imide product.

The alkali metal fluoride is selected from lithium fluoride, sodium fluoride, potassium fluoride or mixtures thereof, and added in an amount 2.01-10 times the moles of lithium bis(chlorosulfonyl)imide.

The saturated alkyl carbonate solvent is selected from dimethyl carbonate, diethyl carbonate, dipropyl carbonate, methyl ethyl carbonate, methyl propyl carbonate, ethyl propyl carbonate or mixtures thereof, and added in an amount 1-10 times the mass fraction of lithium bis(chlorosulfonyl)imide.

According to the invention, the saturated alkyl carbonate solvent may also be a saturated alkyl carbonate based solvent, which is a particular non-protonic mixed solvent, selected from mixtures of two or more of dimethyl carbonate, diethyl carbonate, methyl ethyl carbonate, bis(trifluoroethyl) carbonate, trifluoroethyl methyl carbonate, acetonitrile, tetrahydrofuran, and added in an amount 1-10 times the mass fraction of lithium bis(chlorosulfonyl)imide.

Additionally, the saturated alkyl carbonate based solvent is a saturated fluoroalkyl carbonate based solvent, selected from trifluoroethyl ethyl carbonate, bis(trifluoroethyl) carbonate, trifluoroethyl methyl carbonate or mixtures thereof, and added in an amount 1-10 times the mass fraction of lithium bis(chlorosulfonyl)imide.

The crown ether phase transfer catalyst adaptive to the alkali metal fluoride is 12-crown-4-ether (lithium fluoride), 15-crown-5-ether (sodium fluoride) or 18-crown-6-ether (potassium fluoride).

According to the invention, the amount of the crown ether phase transfer catalyst added is 0.1%-10% of the mass fraction of the starting material lithium bis(chlorosulfonyl)imide.

According to the invention, the temperature of the reaction is 40-130°C and the reaction time is 8-24 hours.

Compared with the prior art preparation method, the invention has the following advantages:
1. The process route is simple and easily controllable;
2. The product can be purified easily to low contents of residual fluorine, chlorine, etc, and thus the quality of the product is stable;
3. The yield is high, and the product purity is so high (≥99.9%) that the requirement of electronic grade can be satisfied.

### Description of Drawing

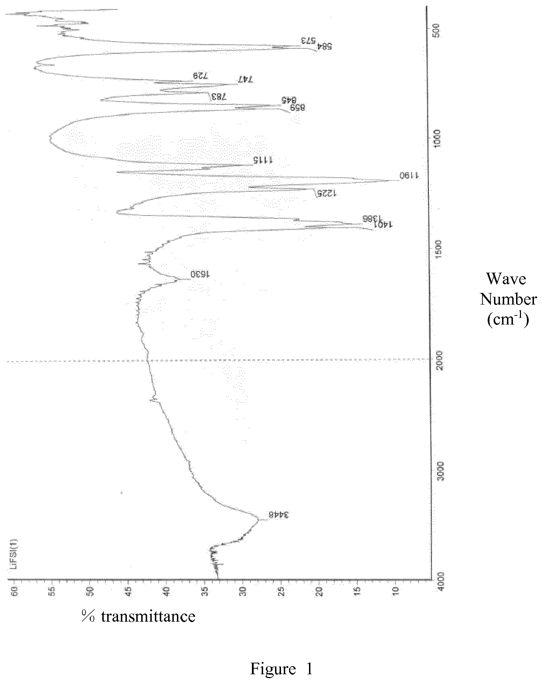

Fig. 1 shows the spectrum of the bis(fluorosulfonyl)imide of the invention measured using Fourier Infrared Spectrometer.

### Detailed Description of the Invention

The invention will be illustrated further in detail with reference to the following specific Examples, so that those skilled in the art can appreciate the advantages of the invention more clearly. Unless otherwise specified, all parts are parts by weight, and all percentages are percentages by weight.

### Example 1

Under the protection of nitrogen gas, to a 1000ml three-neck flask were added 220.0g (1mol) lithium bis(chlorosulfonyl)imide, 220.0g dimethyl carbonate, 52.26g (2mol) lithium fluoride and 0.22g 12-crown-4-ether. After reaction under stirring at 40°C for 8 hours, the resulting mixture was cooled to room temperature and filtered, and the solvent was removed from the filtrate by evaporation to obtain 149g white crystal at a yield of 79.7%. The chlorine content was determined using Swiss Metrohm 848 Potentiometer Titrator to be 7ppm. As measured using Fourier Infrared Spectrometer, 1401cm⁻¹, 1386cm⁻¹, 1190cm⁻¹, 1225cm⁻¹, 859cm⁻¹, 845cm⁻¹, 783cm⁻¹, 747cm⁻¹ correspond to the characteristic groups of bis(fluorosulfonyl)imide by comparing with the standard spectrum of bis(fluorosulfonyl)imide.

### Example 2

Under the protection of nitrogen gas, to a 3000ml three-neck flask were added 220.0g (1mol) lithium bis(chlorosulfonyl)imide, 2200.0g diethyl carbonate, 420.0g (10mol) sodium fluoride and 22g 15-crown-5-ether. After reaction under stirring at 100°C for 24 hours, the resulting mixture was cooled to room temperature and filtered, and the solvent was removed from the filtrate by evaporation to obtain 155g white crystal at a yield of 82.9%. The chlorine content was determined using Swiss Metrohm 848 Potentiometer Titrator to be 5ppm, and the sodium ion content was determined using Shimadzu AA-6300 Atomic Absorption Spectrometer to be 0.7ppm. As measured using Fourier Infrared Spectrometer, 1401cm⁻¹, 1386cm⁻¹, 1192cm⁻¹, 1226cm⁻¹, 859cm⁻¹, 845cm⁻¹, 783cm⁻¹, 747cm⁻¹ correspond to the characteristic groups of bis(fluorosulfonyl)imide by comparing with the standard spectrum of bis(fluorosulfonyl)imide.

### Example 3

Under the protection of nitrogen gas, to a 1000ml three-neck flask were added 220.0g (1mol) lithium bis(chlorosulfonyl)imide, 200.0g diethyl carbonate and 200g dipropyl carbonate, 290.0g (5mol) potassium fluoride and 22g 18-crown-6-ether. After reaction under stirring at 130°C for 14 hours, the resulting mixture was cooled to room temperature and filtered, and the solvent was removed from the filtrate by evaporation to obtain 153g white crystal at a yield of 81.8%. The chlorine content was determined using Swiss Metrohm 848 Potentiometer Titrator to be 2ppm, and the potassium ion content was determined using Shimadzu AA-6300 Atomic Absorption Spectrometer to be 0.2ppm. As measured using Fourier Infrared Spectrometer, 1401cm⁻¹, 1386cm⁻¹, 1190cm⁻¹, 1225cm⁻¹, 859cm⁻¹, 845cm⁻¹, 783cm⁻¹, 747cm⁻¹ correspond to the characteristic groups of bis(fluorosulfonyl)imide by comparing with the standard spectrum of bis(fluorosulfonyl)imide.

### Example 4

Under the protection of nitrogen gas, to a 1000ml three-neck flask were added 220.0g (1mol) lithium bis(chlorosulfonyl)imide, 400.0g bis(trifluoroethyl) carbonate, 290.0g (5mol) potassium fluoride and 22g 18-crown-6-ether. After reaction under stirring at 110°C for 24 hours, the resulting mixture was cooled to room temperature and filtered, and the solvent was removed from the filtrate by evaporation to obtain 156g white crystal at a yield of 83.4%. The chlorine content was determined using Swiss Metrohm 848 Potentiometer Titrator to be 5.1ppm, and the potassium ion content was determined using Shimadzu AA-6300 Atomic Absorption Spectrometer to be 0.6ppm. As measured using Fourier Infrared Spectrometer, 1401cm⁻¹, 1386cm⁻¹, 1190cm⁻¹, 1225cm⁻¹, 859cm⁻¹, 845cm⁻¹, 783cm⁻¹, 747cm⁻¹ correspond to the characteristic groups of bis(fluorosulfonyl)imide by comparing with the standard spectrum of bis(fluorosulfonyl)imide.

### Example 5

Under the protection of nitrogen gas, to a 1000ml three-neck flask were added 220.0g (1mol) lithium bis(chlorosulfonyl)imide, 200.0g bis(trifluoroethyl) carbonate and 200.0g acetonitrile, 290.0g (5mol) potassium fluoride and 22g 18-crown-6-ether. After reaction under stirring at 90°C for 24 hours, the resulting mixture was cooled to room temperature and filtered, and the solvent was removed from the filtrate by evaporation to obtain 152g white crystal at a yield of 81.2%. The chlorine content was determined using Swiss Metrohm 848 Potentiometer Titrator to be 3.1ppm, and the potassium ion content was determined using Shimadzu AA-6300 Atomic Absorption Spectrometer to be 0.3ppm. As measured using Fourier Infrared Spectrometer, 1401cm⁻¹, 1386cm⁻¹, 1190cm⁻¹, 1225cm⁻¹, 859cm⁻¹, 845cm⁻¹, 783cm⁻¹, 747cm⁻¹ correspond to the characteristic groups of bis(fluorosulfonyl)imide by comparing with the standard spectrum of bis(fluorosulfonyl)imide.

### Example 6

Under the protection of nitrogen gas, to a 1000ml three-neck flask were added 220.0g (1mol) lithium bis(chlorosulfonyl)imide, 200.0g bis(trifluoroethyl) carbonate and 200.0g dimethyl carbonate, 232.0g (4mol) potassium fluoride, 26.1g (1mol) lithium fluoride, 18g 18-crown-6-ether and 6g 12-crown-4-ether. After reaction under stirring at 90°C for 24 hours, the resulting mixture was cooled to room temperature and filtered, and the solvent was removed from the filtrate by evaporation to obtain 151g white crystal at a yield of 80.7%. The chlorine content was determined using Swiss Metrohm 848 Potentiometer Titrator to be 3.1ppm, and the potassium ion content was determined using Shimadzu AA-6300 Atomic Absorption Spectrometer to be 0.3ppm. As measured using Fourier Infrared Spectrometer, 1401cm⁻¹, 1386cm⁻¹, 1190cm⁻¹, 1225cm⁻¹, 859cm⁻¹, 845cm⁻¹, 783cm⁻¹, 747cm⁻¹ correspond to the characteristic groups of bis(fluorosulfonyl)imide by comparing with the standard spectrum of bis(fluorosulfonyl)imide.

### Comparative Examples:

According to American Patent US7253317, an alkali metal fluoride and bis(chlorosulfonyl)imide were used to synthesize an alkali metal salt of bis(fluorosulfonyl)imide in nitromethane, wherein the specific examples are as follows:
1. 3.556g (137.1mmol) lithium fluoride was added to 5ml nitromethane, and 4.907g (22.9mmol) bis(chlorosulfonyl)imide was dissolved in 5ml nitromethane. Under stirring, the solution of bis(chlorosulfonyl)imide in nitromethane was added dropwise, followed by reaction overnight after the addition was completed. Supernatant fluid was taken for nuclear magnetic resonance analysis:

| Chemical shift | Peak height | Species |
|---|---|---|
| 56.6 | 1 | FSO₂NH₂ |
| 50.8 | 78.4 | (SO₂F)(SO₂Cl)NH |
| 54.5 | 12.7 | SO₂F |
| 35.3 | 53.3 | FSO₃⁻ |

It was found that no lithium bis(fluorosulfonyl)imide was obtained.
2. 4.421g (29.1mmol) cesium fluoride was added to 2ml nitromethane, and 2.243g (10.48mmol) bis(chlorosulfonyl)imide was dissolved in 5ml nitromethane. Under stirring, the solution of bis(chlorosulfonyl)imide in nitromethane was added dropwise, followed by reaction for 72 hours after the addition was completed.

Supernatant fluid was taken for nuclear magnetic resonance analysis:

| Chemical shift | Peak height | Species |
|---|---|---|
| 56.5 | 15.5 | FSO₂NH₂ |
| 52.1 | 48.3 | (SO₂F)(SO₂Cl)N⁻ |
| 51.9 | 225.6 | (SO₂F)₂N⁻ |

The peak heights in the nuclear magnetic resonance spectrum show that about 77.8% bis(chlorosulfonyl)imide was converted into bis(fluorosulfonyl)imide, but no solid cesium salt of bis(fluorosulfonyl)imide was obtained.

## Claims

1. A method of preparing lithium bis(fluorosulfonyl)imide, comprising: reacting lithium bis(chlorosulfonyl)imide with an alkali metal fluoride selected from lithium fluoride, potassium fluoride, sodium fluoride or mixtures thereof used as a fluorinating agent in a solvent selected from saturated alkyl carbonate and/or saturated fluoroalkyl carbonate in the presence of a crown ether phase transfer catalyst adaptive to the alkali metal fluoride to obtain lithium bis(fluorosulfonyl)imide.

2. The method of preparing lithium bis(fluorosulfonyl)imide according to claim 1, wherein the amount of the alkali metal fluoride added is 2.01-10 times the moles of lithium bis(chlorosulfonyl)imide.

3. The method of preparing lithium bis(fluorosulfonyl)imide according to claim 1 or 2, wherein the saturated alkyl carbonate solvent is selected from dimethyl carbonate, diethyl carbonate, dipropyl carbonate, methyl ethyl carbonate, methyl propyl carbonate, ethyl propyl carbonate or mixtures thereof, and added in an amount 1-10 times the mass fraction of lithium bis(chlorosulfonyl)imide.

4. The method of preparing lithium bis(fluorosulfonyl)imide according to claim 1 or 2, wherein the saturated fluoroalkyl carbonate solvent is selected from trifluoroethyl ethyl carbonate, bis(trifluoroethyl) carbonate, trifluoroethyl methyl carbonate, or mixtures thereof, and added in an amount 1-10 times the mass fraction of lithium bis(chlorosulfonyl)imide.

5. The method of preparing lithium bis(fluorosulfonyl)imide according to claim 1, wherein the crown ether phase transfer catalyst adaptive to the alkali metal fluoride is 12-crown-4(lithium fluoride), 15-crown-5(sodium fluoride) or 18-crown-6(potassium fluoride).

6. The method of preparing lithium bis(fluorosulfonyl)imide according to claim 1 or 4, wherein the amount of the crown ether phase transfer catalyst added is 0.1%-10% of the mass fraction of lithium bis(chlorosulfonyl)imide.

7. The method of preparing lithium bis(fluorosulfonyl)imide according to claim 1, wherein the reaction of the invention is carried out at a temperature of 40-130°C and the reaction time is 8-24 hours.

## Patentansprüche

1. Verfahren zur Herstellung von Lithiumbis(fluorsulfonyl)imid, das Folgendes umfasst: Umsetzen von Lithiumbis(chlorsulfonyl)imid mit einem Alkalimetallfluorid, ausgewählt aus Lithiumfluorid, Kaliumfluorid, Natriumfluorid oder Gemischen davon verwendet als Fluorierungsmittel, in einem Lösemittel, ausgewählt aus gesättigtem Alkylcarbonat und/oder gesättigtem Fluoralkylcarbonat in Gegenwart eines Kronenether-Phasentransferkatalysators, der sich dem Alkalimetallfluorid anpasst, um Lithiumbis(fluorsulfonyl)imid zu erhalten.

2. Verfahren zur Herstellung von Lithiumbis(fluorsulfonyl)imid nach Anspruch 1, wobei die Menge an zugesetztem Alkalimetallfluorid das 2,01-10-fache der Mole an Lithiumbis(chlorsulfonyl)imid beträgt.

3. Verfahren zur Herstellung von Lithiumbis(fluorsulfonyl)imid gemäß Anspruch 1 oder 2, wobei das gesättigte Alkylcarbonat-Lösemittel ausgewählt ist aus Dimethylcarbonat, Diethylcarbonat, Dipropylcarbonat, Methylethylcarbonat, Methylpropylcarbonat, Ethylpropylcarbonat oder Gemischen davon, und in einer 1-10-fachen Menge des Massenanteils von Lithiumbis(chlorsulfonyl)imid zugesetzt wird.

4. Verfahren zur Herstellung von Lithiumbis(fluorsulfonyl)imid nach Anspruch 1 oder 2, wobei das gesättigte Fluoralkylcarbonat-Lösemittel ausgewählt ist aus Trifluorethylethylcarbonat, Bis(trifluorethyl)carbonat, Trifluorethylmethylcarbonat oder Gemischen davon, und in einer 1-10-fachen Menge des Massenanteils von Lithiumbis(chlorsulfonyl)imid zugesetzt wird.

5. Verfahren zur Herstellung von Lithiumbis(fluorsulfonyl)imid nach Anspruch 1, wobei der Kronenether-Phasentransferkatalysator, der sich an das Alkalimetallfluorid anpasst, 12-Krone-4(lithiumfluorid), 15-Krone-5(natriumfluorid) oder 18-Krone-6(kaliumfluorid) ist.

6. Verfahren zur Herstellung von Lithiumbis(fluorsulfonyl)imid nach Anspruch 1 oder 4, wobei die Menge des Kronenether-Phasentransferkatalysators 0,1 bis 10% des Massenanteils von Lithiumbis(chlorsulfonyl)imid beträgt.

7. Verfahren zur Herstellung von Lithiumbis(fluorsulfonyl)imid nach Anspruch 1, wobei die Reaktion der Erfindung bei einer Temperatur von 40-130 °C durchgeführt wird und die Reaktionszeit 8-24 Stunden beträgt.

## Revendications

1. Procédé de préparation de bis(fluorosulfonyl)imide de lithium, comprenant : la mise en réaction du bis (chlorosulfonyl)imide de lithium avec un fluorure de métal alcalin choisi parmi le fluorure de lithium, le fluorure de potassium, le fluorure de sodium ou des mélanges de ceux-ci utilisé comme un agent de fluorisation dans un solvant choisi parmi un carbonate d'alkyle saturé et/ou un carbonate de fluoroalkyle saturé en présence d'un catalyseur de transfert de phase de type éther couronné adapté au fluorure de métal alcalin pour obtenir du bis(fluorosulfonyl)imide de lithium.

2. Procédé de préparation de bis (fluorosulfonyl)imide de lithium selon la revendication 1, dans lequel la quantité du fluorure de métal alcalin ajoutée est 2,01-10 fois la quantité de bis(chlorosulfonyl)imide de lithium en termes de moles.

3. Procédé de préparation de bis(fluorosulfonyl)imide de lithium selon la revendication 1 ou 2, dans lequel le solvant de type carbonate d'alkyle saturé est choisi parmi le carbonate de diméthyle, le carbonate de diéthyle, le carbonate de dipropyle, le carbonate éthylique de méthyle, le carbonate propylique de méthyle, le carbonate propylique d'éthyle ou des mélanges de ceux-ci, et ajouté en une quantité 1-10 fois la fraction massique du bis(chlorosulfonyl)imide de lithium.

4. Procédé de préparation de bis(fluorosulfonyl)imide de lithium selon la revendication 1 ou 2, dans lequel le solvant de type carbonate de fluoroalkyle saturé est choisi parmi le carbonate éthylique de trifluoroéthyle, le carbonate de bis(trifluoroéthyl), le carbonate méthylique de trifluoroéthyle, ou des mélanges de ceux-ci, et ajouté en une quantité 1-10 fois la fraction massique du bis(chlorosulfonyl)imide de lithium.

5. Procédé de préparation de bis (fluorosulfonyl)imide de lithium selon la revendication 1, dans lequel le catalyseur de transfert de phase de type éther couronné adapté au fluorure de métal alcalin est le 12-couronne-4(fluorure de lithium), le 15-couronne-5(fluorure de sodium) ou le 18-couronne-6(fluorure de potassium).

6. Procédé de préparation de bis(fluorosulfonyl)imide de lithium selon la revendication 1 ou 4, dans lequel la quantité du catalyseur de transfert de phase de type éther couronné ajoutée est 0,1 %-10 % de la fraction massique du bis(chlorosulfonyl)imide de lithium.

7. Procédé de préparation de bis(fluorosulfonyl)imide de lithium selon la revendication 1, dans lequel la réaction de l'invention est effectuée à une température de 40-130 °C et le temps de réaction est 8-24 heures.
